Europäisches Patentamt

European Patent Office

Office européen des brevets

(11) Veröffentlichungsnummer: **0 309 882**
**A2**

## (12) EUROPÄISCHE PATENTANMELDUNG

(21) Anmeldenummer: 88115456.1

(22) Anmeldetag: 21.09.88

(51) Int. Cl.⁴: **G01N 33/66 , C12Q 1/26 ,
C12Q 1/34**

(30) Priorität: 29.09.87 DE 3732688

(43) Veröffentlichungstag der Anmeldung:
05.04.89 Patentblatt 89/14

(84) Benannte Vertragsstaaten:
**AT BE CH DE ES FR GB GR IT LI LU NL SE**

(71) Anmelder: **BOEHRINGER MANNHEIM GMBH
Sandhofer Strasse 116
D-6800 Mannheim 31(DE)**

(72) Erfinder: **Vogt, Bernd, Dr.med.
Mozartstrasse 1
D-8132 Tutzing(DE)**
Erfinder: **Schellong, Liselotte
Hallberger Allee 8
D-8132 Tutzing(DE)**
Erfinder: **Siedel, Joachim, Dr.rer.nat.
Weilheimer Strasse 10
D-8139 Bernried(DE)**
Erfinder: **Ziegenhorn, Joachim, Dr.rer.nat.
Ina-Seidel-Weg 1
D-8130 Starnberg(DE)**

(54) **Verfahren zur spezifischen Bestimmung des Serumfructosamingehalts sowie hierfür geeignetes Reagenzgemisch.**

(57) Die vorliegende Erfindung betrifft ein Verfahren zur spezifischen Bestimmung des Serumfructosamingehaltes in Blut oder von Blut abgeleiteten Proben durch Umsetzung mit einem geeigneten Farbreagenz und Messung der dabei bewirkten Farbänderung. Das erfindungsgemäße Verfahren ist dadurch gekennzeichnet, daß vor der Farbreaktion unspezifisch reduzierend wirkende oder/und trübungsverursachende Probenbestandteile bei ungefähr neutralem pH-Wert beseitigt werden, anschließend ein pH-Wert zwischen pH 10 und 12 eingestellt und das Farbreagenz zugegeben wird.

Ein weiterer Gegenstand der Erfindung ist ein Reagenzkombination zur spezifischen Bestimmung des Serumfructosamingehaltes in Blut oder von Blut abgeleiteten Proben, welche dadurch gekennzeichnet ist, daß sie aus einem Reagenz zur Beseitigung unspezifisch reduzierend wirkender oder/und trübungsverursachender Probenbestandteile, einem Umpufferungsreagenz mit einem alkalischen Puffer und einem Farbreagenz zum Nachweis des Fructosamins besteht.

EP 0 309 882 A2

Fig. 1

HPLC Referenzmethode (relative Peakflächeneinheiten)

x Normalseren          O Diabetikerseren

Anzahl Wertepaare:       33

Korrelationskoeffizient: 0.945

Standard-Hauptkomponenten-Analyse: $Y = 1.41 + 0.000748 \times X$

## Verfahren zur spezifischen Bestimmung des Serumfructosamingehalts sowie hierfür geeignete Reagenzkombination

Die vorliegende Erfindung betrifft ein Verfahren zur spezifischen Bestimmung des Serumfructosamingehalts in Blut oder von Blut abgeleiteten Proben, bei dem störende Probenbestandteile vor Messung des Fructosamingehaltes beseitigt werden.

Unter Serumfructosamingehalt versteht man den Gesamtgehalt an nichtenzymatisch glycosilierten Serumproteinen. Diese entstehen dadurch, daß Serumglucose über ihre Carbonylgruppe mit freien Proteinaminoresten Schiff'sche Basen bildet. Diese gehen anschließend durch Amadori-Umlagerung in Fructosamine mit stabiler Ketoamin-Bindung über.

Die Halbwertzeit des Serumfructosamins ist wegen der Stabilität der Ketoamin-Bindung praktisch identisch mit der der Serumproteine, deren Halbwertzeit im Durchschnitt ca. 21 Tage beträgt. Entsprechende Untersuchungen wurden von L. Y. Seng und M. J. Staley in J. Clin. Chem. (1986) 32, 560 veröffentlicht.

Das Ausmaß der Fructosamin-Bildung ist proportional dem Blutglucose-Spiegel. Dieser kann bekanntermaßen bei Diabetikern, besonders bei ungenügender diätetischer und medikamentöser Stoffwechseleinstellung starken Schwankungen, verbunden mit ausgeprägten pathologischen Erscheinungen, unterliegen.

Die Blutglucose-Bestimmung gibt dem Arzt nur Auskunft über die zum Zeitpunkt der Blutentnahme vorhandene Stoffwechsellage. Eine Langfristkontrolle über die Stoffwechsellage der letzten 120 Tage ist dagegen mit der Bestimmung des glycosilierten Hämoglobins (HbA$_1$) möglich. Die Messung des Serumfructosamins eignet sich nun gerade wegen dessen Halbwertzeit dazu, die Stoffwechselführung von Diabetikern durch Lebenshaltung und therapeutische Maßnahmen rückwirkend über einen mittelfristigen Zeitraum von ca. 3 Wochen zu ermitteln. In Verbindung mit den etablierten klinisch diagnostischen Parametern Blutglucose sowie glycosiliertes Hämoglobin (HbA$_1$) ließe sich deshalb mit einer zuverlässigen, spezifischen und praktikablen Methode zur Serumfructosamin-Bestimmung das Diagnosearsenal zur Überwachung von Diabetikern um einen wertvollen Mittelfristparameter erweitern.

Ein vom Prinzip her einfach durchführbares Verfahren zur Serumfructosamin-Bestimmung wurde von Baker in EP-C 0 085 263 und von Johnson et al. in Clin. Chim. Acta (1982) 127, 87 - 95 beschrieben. Es beruht darauf, daß die Ketoaminform in wässrigem, alkalischem Medium in eine Enolform übergeht, die reduzierend auf Tetrazoliumsalze, wie z..B. Nitrotetrazoliumblau wirkt und dabei einen Formazanfarbstoff liefert. Das Ausmaß der in einem definierten Zeitintervall bei 37° C photometrisch gemessenen Farbstoffbildung sollte der Menge an vorliegendem Fructosamin proportional sein.

Dieser Test ist bei Verwendung von Serum als Probenmaterial störanfällig, denn natürliche Serumbestandteile, wie Bilirubin, Harnsäure und SH-Gruppen, wirken ebenfalls auf Tetrazoliumsalze reduktiv. Auch Medikamente, wie z. B.α -Methyldopa, Medikamentabbauprodukte, wie z. B. Gentisinsäure, die ein Metabolit der Acetylsalicylsäure ist, sowie Ascorbinsäure führen je nach Konzentration im Serum zu verfälschten Meßergebnissen.

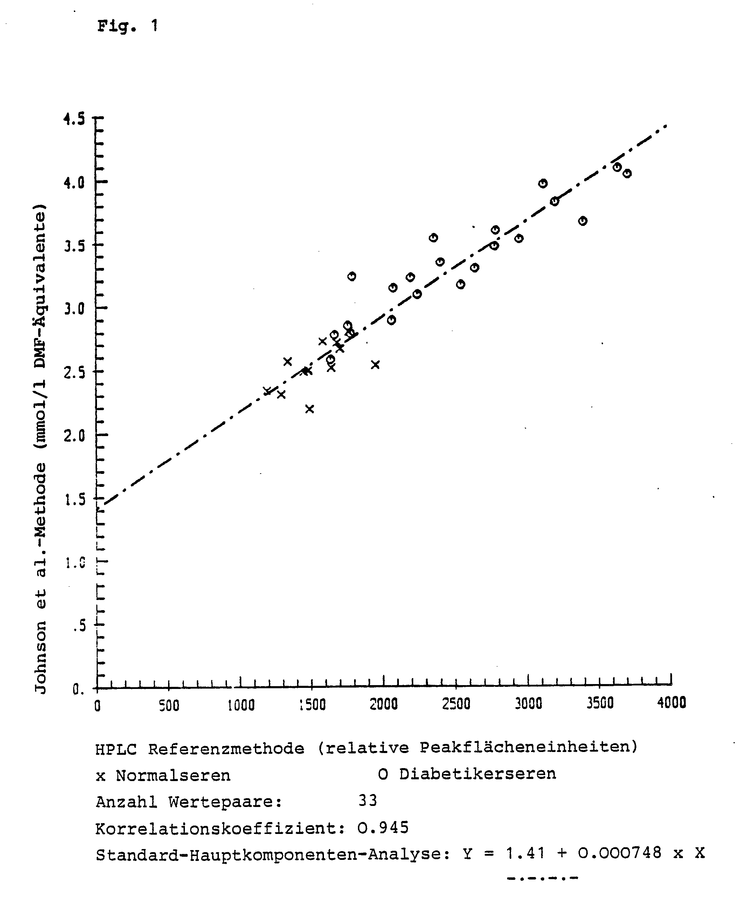

Diese Störungen wirken sich bereits bei ansonsten unauffälligen normalen und diabetischen Seren so aus, daß bei einem Methodenvergleich z. B. mit der Furosin/HPLC-Methode nach Schleicher und Wieland (J. Clin. Chem. Clin. Biochem. 19, 81-87 (1981), die Absolutangaben über das Ausmaß der nichtenzymatischen Glykosilierung ermöglicht, und dem Verfahren zur Serumfructosaminbestimmung nach Baker sich zwar eine lineare Korrelation des Fructosamingehalts ergibt, die Baker-Methode jedoch dann bereits eine beträchtliche nichtenzymatische Glykosilierung anzeigt, wenn dies tatsächlich noch gar nicht der Fall ist. Schematisch wirkt sich dies in einem Diagramm in dem die Ergebnisse der Baker-Methode gegen diejenigen der Furosin/HPLC-Methode aufgetragen werden, so aus, daß auf Seiten des Baker-Tests bereits bei völliger Abwesenheit von Serumfructosamin ein Achsenabschnitt auftritt, dessen Wert etwa 50 % desjenigen durchschnittlichen Nichtdiabetiker ausmacht (vgl. Fig. 1).

Serumfructosamin-Bestimmungen leiden bisher außerdem unter Störungen, die auf dem von Probe zu Probe variierenden Gesamtproteingehalt beruhen. Sie führen zu Meßwertschwankungen und verringern dadurch die Empfindlichkeit des Bestimmungsverfahrens. Diese Störungen sind als Matrixeffekte bekannt. Besonders bemerkbar macht sich dieser Effekt bei Zugabe zusätzlichen Proteins, wie dies z. B. bei der Herstellung von Standardlösungen üblicherweise der Fall ist. Steigende Proteinmengen verlangsamen die Reaktion zwischen Fructosamin und Farbreagenz (vergleiche E. J. Hindle et al., Ann. Clin. Biochem. 22 - (1985), S. 84 - 89).

Weitere Schwierigkeiten treten bei der Fructosamin-Bestimmung in hyperlipämischen Seren auf. Im allgemeinen ist, um ein auch bei niedrigen Fructosaminkonzentrationen in der Probe noch ausreichend großes Meßsignal zu erhalten, ein Probe-/Reagenzvolumenverhältnis von 0,1 erforderlich. Bei überhöhten Triglyceridkonzentrationen macht sich jedoch bei einem so hohen Probenanteil die resultierende Trübung des Testansatzes negativ bei der photometrischen Messung bemerkbar. Die Fructosamin-Bestimmung wird erheblich erschwert oder sogar verhindert.

Es besteht deshalb weiterhin Bedarf an einem Verfahren zur spezifischen Bestimmung des Serumfructosamingehaltes in Blut oder von Blut abgeleiteten Proben, das die oben genannten Nachteile nicht aufweist. Aufgabe der vorliegenden Erfindung war es, ein solches Verfahren bereitzustellen.

Gelöst wird diese Aufgabe durch die in den Patentansprüchen gekennzeichnete Erfindung. Das erfindungsgemäße Verfahren zur spezifischen Bestimmung des Serumfructosamingehaltes in Blut oder von Blut abgeleiteten Proben durch Umsetzung mit einem geeigneten Farbreagenz und Messung der dadurch bewirkten Farbänderung, ist dadurch gekennzeichnet, daß vor der Farbreaktion unspezifisch reduzierend wirkende oder/und trübungsverursachende Probenbestandteile bei ungefähr neutralem pH-Wert beseitigt werden, anschließend ein pH-Wert zwischen 10 und 12 eingestellt und das Farbreagenz zugegeben wird.

Zur Beseitigung unspezifisch reduzierend wirkender Probenbestandteile wird die Probe mit einem oder mehreren oxidierend wirkenden Enzymen versetzt. Vorteilhaft werden als enzymatische Oxidationsmittel Ascorbat-Oxidase, Bilirubin-Oxidase oder/und Uricase zugesetzt. Peroxidase kann mit gegebenenfalls entstehendem Wasserstoffperoxid zusätzliche oxidative Abbauprozesse bewirken, was die Wirksamkeit des oder der Oxidationsmittel noch zusätzlich erhöht. Gegebenenfalls zugesetzte Katalase dient vor allem zur Beseitigung überschüssigen Wasserstoffperoxids.

Insbesondere für die Untersuchung lipämischer Proben hat es sich als zweckmäßig erwiesen, dem oder den oxidierend wirkenden Enzymen gegebenenfalls Lipase, gegebenenfalls zusammen mit einem oder mehreren Detergentien oder/und Salzen starker Säuren zuzusetzen. Mit Hilfe eines solchen Gemisches gelingt es, trübungsverursachende Stoffe soweit zu eliminieren, daß die anschließende Farbmessung nicht mehr beeinträchtigt ist. Je nach Probenverdünnung und Detergenzienzugabe kann die Lipämiestörung aber auch ausschließlich nichtenzymatisch mittels einem oder mehreren Detergenzien beseitigt werden.

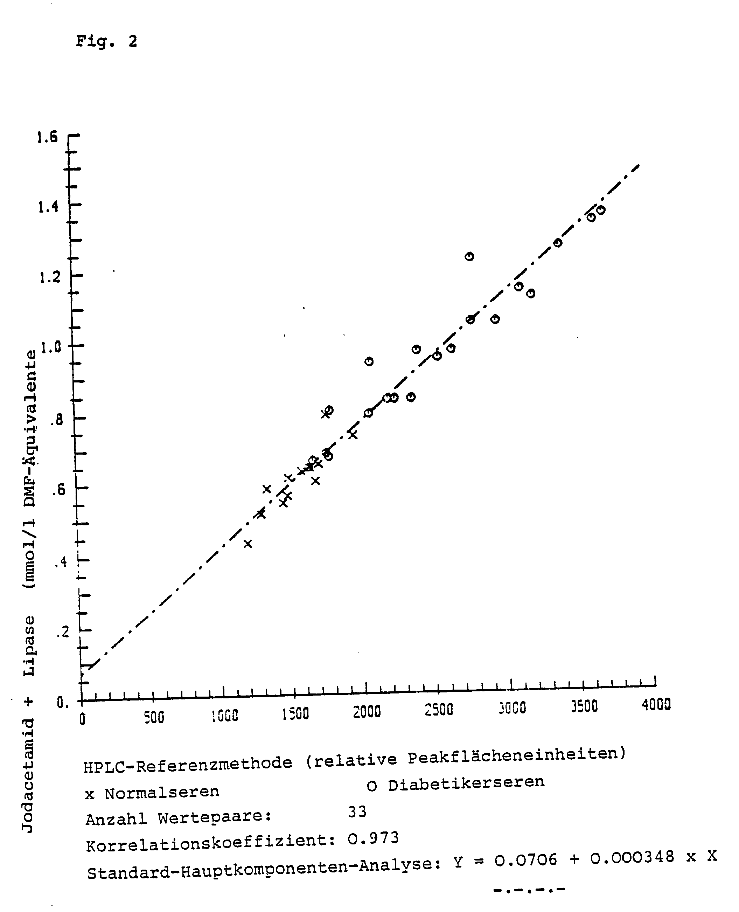

Überraschenderweise hat es sich gezeigt, daß durch weiteren Zusatz von einem oder mehreren SH-Gruppen blockierenden Reagenzien insgesamt eine gute Korrelation der erfindungsgemäßen Serumfructosaminbestimmungsmethode mit der Furosin/HPLC-Vergleichsmethode ohne einen Achsenabschnitt erreicht wird. Bei Abwesenheit von nichtenzymatisch glycosilierten Proteinen in der Probe wird so also tatsächlich auch keine Farbreaktion beobachtet (Fig. 2).

SH-Gruppen blockierende Reagenzien sind solche, die mit SH-Gruppen reagieren. Sie sind in der einschlägigen Literatur zahlreich beschrieben. Es sei auf folgende Übersichtarbeiten verwiesen:

A.N. Glazer, R.J. Delange, D.S. Sigman; Chemical modification of proteins. Laboratory techniques in biochemistry and molecular biology (T.S. Work and E. Work, eds.) (1975).

C.H.W. Hirs, S.N. Timasheff, eds.; Modification reactions. In: Methods in Enzymology 25, 387-671 (1972).

C.H.W. Hirs, S.N. Timasheff, eds.; Chemical modification. In: Methods in Enzymology 47, 407-500 (1977).

R.L. Lundblad, C.M. Noyes, Chemical reagents for protein modification. CRC Press, Boca Rotan, Florida (1984).

Gängige SH-Blocker sind z. B. Jodacetamid, Jodacetat, N-Ethylmaleinimid, p-Hydroxymercuribenzoat. Als besonders geeignet für das erfindungsgemäße Verfahren erwies sich Jodacetamid.

Mit Hilfe der vorgenannten Substanzen zur Beseitigung störender Bestandteile werden auch die als Matrixeffekte bekannten Meßwertschwankungen durch variierende Gesamtproteinmengen in der Probe weitgehend unterdrückt bzw. vollständig beseitigt. Damit ist die Voraussetzung für eine richtige Protein-Korrektur durch Bildung des Quotienten Fructosamin/Gesamtprotein gegeben.

Zur Beseitigung unspezifisch reduzierend wirkender oder/und trübungsverursachender Probenbestandteile hat es sich als zweckmäßig erwiesen, die Probe mit einer Lösung zu inkubieren, welche oxidierend wirkende sowie gegebenenfalls ein oder mehrere lipidspaltende Enzyme, SH-Gruppen blockierende Substanzen, sowie gegebenenfalls ein oder mehrere Detergentien oder/und Salze starker Säuren in einem geeigneten nichtreduzierenden Puffer enthält. Zur Herstellung dieser Lösung sind sämtliche Puffersubstanzen geeignet, die selbst nicht reduzierend wirken und deren Pufferwirkung bei ungefähr neutralem pH-Wert liegt. Vorteilhaft hat sich ein pH- Bereich von pH 6 - 9, vorzugsweise pH 7 - 8,5, ganz besonders bevorzugt pH 7,5 - 8,0, erwiesen. Die Pufferkonzentration beträgt vorzugsweise 10 - 100 mmol/l, ganz besonders bevorzugt 20 - 70 mmol/l. Als besonders vorteilhaft hat sich wässriger Kaliumphosphatpuffer erwiesen.

Die Konzentration der zugesetzten Enzyme richtet sich nach der Konzentration der zu beseitigenden störenden Verbindungen. Üblicherweise liegen diese Enzymkonzentrationen zwischen 0,01 und 10 000 U/ml. Bevorzugte Konzentrationsbereiche sind beispielsweise für

| Uricase | 1 | - | 15 U/ml |
|---|---|---|---|
| Bilirubin-Oxidase | 0,05 | - | 5 U/ml |
| Ascorbat-Oxidase | 2 | - | 20 U/ml |
| Lipase | 0,5 | - | 5 U/ml |
| Peroxidase | 0,5 | - | 5 U/ml |
| Katalase | 100 | - | 10 000 U/ml |

Besonders bevorzugte Konzentrationsbereiche dieser Enzyme sind für

| Uricase | 2 | - | 10 U/ml |
|---|---|---|---|
| Bilirubin-Oxidase | 0,1 | - | 1 U/ml |
| Ascorbat-Oxidase | 5 | - | 15 U/ml |
| Lipase | 1 | - | 3 U/ml |
| Peroxidase | 1 | - | 3 U/ml |
| Katalase | 500 | - | 2000 U/ml. |

Als SH-Gruppen blockierende Substanzen sind prinzipiell alle diesbezüglich literaturbekannten Reagenzien geeignet, z. B. Jodacetamid, Jodacetat, N-Ethylmaleinimid und p-Hydroxymercuribenzoat. Als besonders geeignet erwies sich Jodacetamid. Als bevorzugter Konzentrationsbereich für SH-Blocker erwies sich 0,5 - 500 mmol/l, besonders bevorzugt 5 - 50 mmol/l.

Detergentien können außer kationischen auch anionische oder nichtionische Detergentien sein. Als anionische Detergentien werden Alkali- oder Erdalkalisalze von Gallensäuren und ihrer Konjugate bevorzugt. Vorteilhafte Konzentrationen anionischer Detergentien liegen zwischen 2 und 10 mmol/l. Als besonders günstig hat sich Natriumcholat in Konzentrationen von 4 bis 6 mmol/l erwiesen.

Als nichtionische Detergentien steht eine breite Palette von Detergentien zur Auswahl. Als geeignet haben sich vor allem lineare oder verzweigtkettige Alkyl- oder Alkylaryl-Alkohol-Polyglycoläther mit 8 bis 20 C-Atomen im Alkoholteil und 4 bis 15 Glycoleinheiten pro Molekül erwiesen. Eine besonders vorteilhafte aktivierende Wirkung üben lineare und verzweigtkettige Alkyl-Alkohol-Polyglycoläther mit 8 bis 12 C-Atomen im Alkoholteil und 4 bis 8 Glycoleinheiten pro Molekül aus.

Für das erfindungsgemäße Verfahren zur spezifischen Bestimmung des Serumfructosamingehaltes sind nichtionische Detergentien geeignet, die bezüglich der Struktur des Alkoholteils einheitlich oder ein Gemisch aus mehreren, hinsichtlich der Struktur des Alkoholteils unterschiedlichen Polyglycoläthern sein können. Geeignete nichtionische Detergenzien sind z. B. Oxetal® ID 104 (Zschimmer & Schwarz, Lahnstein, Bundesrepublik Deutschland), Produkt RT 240® (Zschimmer & Schwarz, Lahnstein, Bundesrepublik Deutschland), Lutensol® ON 50, ON 60, ON 70 (BASF, Ludwigshafen, Bundesrepublik Deutschland) sowie Soprofor® D/916 (Rhone-Poulenc, Frankreich). Die Konzentration an nichtionischem Detergenz, die erfindungsgemäß zur Beseitigung unspezifisch reduzierend wirkender oder/und trübungsverursachender Probenbestandteile eingesetzt wird, reicht von 0,05 bis 15 Gew. %. Als bevorzugter Konzentrationsbereich hat sich 0,1 bis 5 Gew.-% erwiesen.

Die trübungsbeseitigende Wirkung kann durch höhere Ionenstärken in der Reaktionslösung noch verstärkt werden. Hierfür haben sich Zusätze von Salzen starker Säuren, die auch im alkalischen pH-Bereich in Lösung bleiben, als günstig erwiesen. Bevorzugt werden Alkali- oder Erdalkalisalze von Salz- oder Schwefelsäure. Besonders bevorzugt werden Kalium- oder Natriumchlorid eingesetzt. Die Konzentration der zugesetzten Salze starker Säuren reichen von 20 bis 100 mmol/l. Besonders bevorzugt werden Salze in der Konzentration von 40 bis 60 mmol/l zugesetzt.

Die Beseitigung unspezifisch reduzierend wirkender oder/und trübungsverursachender Probenbestandteile erfolgt bei Temperaturen zwischen 25 und 40° C, vorzugsweise bei 37° C über einen Zeitraum von 1 bis 15 Minuten, vorzugsweise 2 bis 6 Minuten. Die zu wählende Zeitspanne der Inkubation ist abhängig von der Menge an unspezifisch reduzierend wirkenden und trübungsverursachenden Probenbestandteilen und der zu ihrer Beseitigung eingesetzten Menge an Zusätzen.

Da die Inkubation zur Beseitigung unspezifisch reduzierend wirkender oder/und trübungsverursachender Probenbestandteile wegen des pH-Optimums der verwendeten Enzyme bei ungefähr neutralem pH-Wert durchgeführt wird, die Farbreaktion zwischen Farbreagenz und Fructosamin aber bei einem pH-Wert zwischen 10 und 12 verläuft, ist es erforderlich, nach erfolgter Inkubation umzupuffern. Die Erhöhung des pH-Wertes erfolgt mittels eines Puffers, dessen pH-Wert etwas über dem einzustellenden pH-Wert liegt. Ein Puffer mit einem pH-Wert zwischen pH 10,5 und 12,5, besonders bevorzugt zwischen pH 10,7 und 12,2 ist

4

besonders zweckmäßig. Vorteilhafterweise wird hierfür ein Carbonatpuffer eingesetzt, dessen Konzentration 150 bis 300 mmol/l, besonders bevorzugt 180 bis 220 mmol/l beträgt.

In an sich bekannter Weise kann durch Zugabe eines Farbreagenzes im alkalischen Bereich die reduzierende Wirkung des Fructosamins sichtbar gemacht werden. Vorzugsweise wird hierfür ein Tetrazoliumsalz eingesetzt, dessen Formazanfarbstoffbildung visuell oder photometrisch verfolgt werden kann. Als Tetrazoliumsalze werden diejenigen bevorzugt, die in "Methods of Enzymatic Analysis" (H. U. Bergmeyer, Hrsg., 3. Ausgabe, Verlag Chemie Weinheim 1983, Band I, Seite 200) beschrieben sind. Besonders geeignet sind Nitrotetrazoliumblau (NBT) oder 3-(4´,5´-Dimethylthiazolyl-2-)-2,4-diphenyl-tetrazoliumbromid (MTT). Das Farbreagenz kann sowohl nach der Umpufferung als auch gleichzeitig mit dem Puffer dem Testansatz zugegeben werden. Hierfür hat es sich als vorteilhaft erwiesen, das Farbreagenz in dem für die Umpufferung benötigten Puffer zu lösen. Für Tetrazoliumsalze haben sich Konzentrationen von 0,2 bis 2 mmol/l als günstig, 0,4 bis 1,5 mmol/l als besonders günstig erwiesen.

Zur Umpufferung nach der Beseitigung unspezifisch reduzierend wirkender oder/und trübungsverursachender Probenbestandteile wird soviel Puffer zugesetzt, daß der pH-Wert des Testansatzes bei Anwesenheit des Farbreagenzes einen pH-Wert zwischen 10 und 12, vorzugsweise 10,3 bis 10,6 augweist.

Die umgepufferte Lösung wird bei Temperaturen zwischen 25 und 40° C, vorzugsweise bei 37° C inkubiert. Die Farbänderung beruhend auf der Reduktion des Farbreagenzes wird photometrisch in einem definierten Zeitintervall, vorzugsweise 1 bis 15 Minuten nach Umpufferung verfolgt. Eine erste Messung wird 1 bis 10 Minuten nach der Umpufferung und eine letzte Messung 2 bis 15 Minuten nach der Umpufferung durchgeführt. Je nach Erfordernis können zwei oder mehr Messungen durchgeführt werden. Die zeitlichen Abstände zwischen zwei Messungen sind variabel. Sie können je nach apparativer Ausstattung gewählt werden, wobei sie im Bereich weniger Sekunden als auch einiger Minuten liegen können.

Es ist zweckmäßig, die erhaltenen Meßwerte mit denen einer Standardlösung zu vergleichen. Geeignete Standardlösungen sind bekannt. Beispielsweise kann hierzu der von Johnson et al. in Clin. Chim. Acta (1982) 127, 87 - 95 beschriebene Standard verwendet werden, der auf einer Matrix aus Humanalbumin mit definierten Zusätzen eines synthetischen Fructosamins basiert. Als synthetisches Fructosamin wird I-Desoxy-I-morpholino-fructose (DMF) verwendet. Die ermittelte Serumfructosamin-Konzentration in der Probe wird bei Verwendung dieses Standards in DMF-Einheiten angegeben. Ebenso geeignet als Standard ist ein Humanserumalbumin oder Rinderserumalbumin oder andere Proteine mit jeweils bekanntem Glykosylierungsgrad.

Überraschenderweise erlaubt es das erfindungsgemäße Verfahren auch, das Probe-/Reagenzvolumenverhältnis von 0,1, das nach dem Verfahren von Johnson et al. üblich ist, stark herabzusetzen, ohne daß bei gleichem Meßintervall und Inkubation bei gleicher Temperatur das Meßsignal mit einer definierten Menge an einem als Probe eingesetzten Fructosaminanalogon signifikant im Vergleich zu der von Johnson et al. beschriebenen Methode kleiner wird. Wird als Fructosaminanalogon z. B. DMF verwendet, so kann in diesem Fall das Probe-/Reagenzvolumenverhältnis auf 0,02 herabgesetzt werden, ohne die Empfindlichkeit der Messung zu verringern.

Das erfindungsgemäße Verfahren zur Bestimmung von Fructosamin in Blut oder von Blut abgeleiteten Proben, wobei unspezifisch reduzierend wirkende oder/und trübungsverursachende Probenbestandteile mit Hilfe eines oder mehrerer Enzyme und SH-Blocker sowie gegebenenfalls einem oder mehreren Detergentien oder/und Salzen beseitigt werden, läßt sich nicht nur in Lösung durchführen. Es ist auch ohne weiteres auf Bestimmungsverfahren mittels trockenchemischer Testträger übertragbar. Die Enzyme und SH-Blocker sowie gegebenenfalls ein oder mehrere Detergentien oder/und Salze eventuell mit weiteren Hilfsstoffen werden hierzu in an sich bekannter Weise auf feste Träger aufgebracht. Geeignete feste Träger sowie Verfahren zum Aufbringen dieser Stoffe bzw. Stoffgemische auf solche Träger sind dem Fachmann bekannt. Als Trägermaterialien eignen sich beispielsweise alle möglichen saugfähigen Materialien, wie beispielsweise Papiere, Vliese usw.. Die aufzubringenden Stoffe können in eine oder mehrere Imprägnierlösungen aufgenommen werden. Mit diesen Lösungen werden die Träger imprägniert oder besprüht. Anschließend wird getrocknet.

Eine andere Möglichkeit wäre, die Enzyme und SH-Blocker sowie gegebenenfalls Detergentien oder/und Salze sowie eventuell weitere Hilfsstoffe in Reagenzfilme einzubringen. Hierzu werden die Substanzen bzw. Substanzgemische z. B. entsprechend Verfahren gemäß DE-PS 1 598 153 oder DE-OS 2 910 134 zu Reagenzfilmen verarbeitet.

Zur Beseitigung störender unspezifisch reduzierend wirkender oder/und trübungsverursachender Probenbestandteile wird die zu messende Probe zunächst mit einem Träger in Verbindung gebracht, der das oder die Enzyme und SH-Gruppen blockierenden Reagenzien sowie gegebenenfalls ein oder mehrere Detergentien oder/und Salze enthält. Nach ausreichendem Kontakt wird die vorbehandelte Probe in eine Schicht überführt, welche die weiteren zur Farbreaktion erforderlichen Reaktionsbestandteile enthält. Die dabei bewirkte Farbänderung wird in bekannter Weise photometrisch, z. B. reflektometrisch gemessen. In Bezug auf die Zeitintervalle für die Vorreaktion und für die Farbreaktion gelten die oben gemachten Ausführungen.

Ein weiterer Gegenstand der Erfindung ist eine Reagenzkombination zur spezifischen Bestimmung des Serumfructosamingehaltes in Blut oder von Blut abgeleitete Proben, welche dadurch gekennzeichnet ist, daß sie aus einem Reagenz zur Beseitigung unspezifisch reduzierand wirkender oder/und trübungsverursachender Probenbestandteile, einem Umpufferungsreagenz mit einem Puffer, der einen pH-Wert im Bereich von 10,5 - 12,5 aufweist und einem Farbreagenz zum Nachweis des Fructosamins besteht. Diese Reagenzkombination enthält alle zur Durchführung des erfindungsgemäßen Verfahrens notwendigen Bestandteile.

Das Reagenz zur Beseitigung unspezifisch reduzierend wirkender oder/und trübungsverursachender Probenbestandteile in Blut oder von Blut abgeleiteten Proben besteht aus einem oder mehreren enzymatischen Oxidationsmitteln sowie gegebenenfalls Peroxidase oder/und Katalase oder/und Lipase, einem oder mehreren SH-Gruppen blockierenden Reagenzien sowie gegebenenfalls einem oder mehreren Detergentien oder/und Salzen starker Säuren sowie gegebenenfalls üblichen Zusatzstoffen in einem Puffer mit annähernd neutralem pH-Wert.

Unter enzymatischen Oxidationsmitteln sind insbesondere Ascorbat-Oxidase, Bilirubin-Oxidase und Uricase zu verstehen.

SH-Gruppen blockierende Reagenzien sind insbesondere Jodacetamid, Jodacetat, N-Ethylmaleinimid, p-Hydroxymercuribenzoat und zu diesen Substanzen verwandte Verbindungen.

Die in dem erfindungsgemäßen Reagenz zur Beseitigung unspezifisch reduzierend wirkender oder/und trübungsverursachender Probenbestandteile in Blut oder in von Blut abgeleiteten Proben gegebenenfalls enthaltenen Detergentien können anionisch oder nichtionisch sein. Während als anionische Detergentien vor allem Alkali- oder Erdalkalisalze von Gallensäuren und ihrer Konjugate bevorzugt werden, steht für nichtionische Detergentien eine breite Palette von Detergentien zur Auswahl. Als geeignet haben sich vor allem lineare oder verzweigtkettige Alkyl- oder Alkylaryl-Alkohol-Polyglycoläther mit 8 - 20 C-Atomen im Alkoholteil und 4 bis 15 Glycoleinheiten pro Molekül erwiesen. Die zugesetzten Detergentien können jedoch zusätzlich auch kationische Detergentien sein, wie beispielsweise Oxyethylalkylammoniumphosphat (z. B. Dehyquart®SP der Fa. Henkel, Düsseldorf, Bundesrepublik Deutschland).

Ganz besonders wirksam ist ein Gemisch enthaltend die Enzyme Peroxidase und Uricase, den SH-Blocker Jodacetamid, ein nichtionisches Detergenz, wie z. B. Lutensol® ON 60 sowie das anionische Detergenz Cholat.

Als Salze starker Säuren haben sich für das erfindungsgemäße Reagenz Alkali- oder Erdalkalisalze von Salz- oder Schwefelsäure als geeignet gezeigt. Besonders bevorzugt werden Kalium- oder Natriumchlorid.

Der zur Erzielung eines annähernd neutralen pH-Wertes des erfindungsgemäßen Reagenzes erforderliche Puffer weist einen pH-Wert in dem Bereich von pH 6 - 9, vorzugsweise pH 7 - 8,5, ganz besonders bevorzugt pH 7,5 - 8,0 auf. Die Pufferkonzentration beträgt vorzugsweise 10 - 100 mmol/l, ganz besonders bevorzugt 20 - 70 mmol/l. Als besonders vorteilhaft hat sich wässriger Kaliumphosphatpuffer erwiesen.

Erfindungsgemäßes Verfahren und Reagenzkombination sind in den folgenden Beispielen weiter erläutert.

## Beispiel 1

Bestimmung von Fructosamin in Serum nach der Methode von Johnson et al. am Analysenautomaten.

## Reagenzzusammensetzung:

0,1 mol/l Natriumcarbonatpuffer pH 10,35
0,25 mmol/l Nitrotetrazoliumblau

## Durchführung der Bestimmung:

20 µl Probe werden mit 200 µl Reagenz und 50 µl $H_2O$ (Diluent) vermischt und bei 37 °C an einem Cobas Bio® Analysenautomaten (Hoffmann-La Roche, Basel, Schweiz) bei = 530 nm die Extinktion zeitabhängig bestimmt. Zur Ermittlung der Fructosaminkonzentration wird die Kinetik zwischen der 10. und 15. Minute nach Zugabe des Reagenzes ausgewertet.

Diese Ergebnisse wurden gegen die Meßwerte aufgetragen, welche mit der Furosin/HPLC-Bezugsmethode (J. Clin. Chem., Clin. Biochem. 19 (1981, 81-87) erhalten wurden. Als HPLC-Meßwerte werden jeweils relative Peakflächeneinheiten verwendet (Fig. 1).

Auch bei den nachfolgenden Beispielen wird die Furosin/HPLC-Methode als Referenzmethode verwendet.

Beispiel 2

Bestimmung von Fructosamin in Serum nach dem erfindungsgemäßen Verfahren am Analysenautomaten.

A) Reagenzienzusammensetzung

| a) Reagenz I | |
|---|---|
| Komponente | Konzentration |
| Kaliumphosphatpuffer pH 8,0 | 50 mMol/l |
| Kaliumchlorid | 50 mMol/l |
| Natriumcholat | 5,6 mMol/l |
| Lutensol® ON60 (BASF, Ludwigshafen, BRD) | 2,5 % (Gew) |
| Uricase | 4 U/ml |
| Peroxidase | 2 U/ml |
| Lipase | 2 U/ml |
| Jodacetamid | 20 mMol/l |

| b) Reagenz II | |
|---|---|
| Komponente | Konzentration |
| Natriumcarbonatpuffer pH 10,9 | 200 mMol/l |
| Nitroblautetrazoliumsalz (NBT) | 0,5 mMol/l |

B) Testdurchführung

Die Tests werden an einem Hitachi 704 Analysenautomaten durchgeführt.

Wellenlänge: 546 nm
Temperatur: 37°C
Schichtdicke: 10 mm (Halbmikroküvette)

Es werden in Küvetten pipettiert:

| | Probe (P) | Reagenzleerwert (RL) |
|---|---|---|
| Reagenz I | 175 μl | 175 μl |
| Probe | 7 μl | - |
| dest. Wasser | - | 7 μl |

Es wird 5 Minuten bei 37°C inkubiert. Dann wird zugemischt:

| Reagenz II | 175 μl | 175 μl |
|------------|--------|--------|

Es wird erneut inkubiert und die Kinetik der Farbstoffbildung ($\Delta E_p$ bzw. $\Delta E_{RL}$) innerhalb eines bestimmten Zeitintervalls $\Delta t$ (8 bis 10 Minuten) gemessen.

In entsprechender Weise wie vorstehend für die Probe ausgeführt, wird eine Standardlösung (S) mit bekanntem Fructosamingehalt vermessen und aus den so erhaltenen Werten mittels Dreisatz die Fructosaminkonzentration der Probe errechnet.

In Fig. 2 sind die so erhaltenen Meßwerte gegen solche, die mit der Furosin/HPLC-Methode ermittelt wurden, aufgetragen.

Ein Vergleich der Ergebnisse von Beispiel 1 und Beispiel 2 zeigt, daß mit dem erfindungsgemäßen Verfahren gegenüber dem Stand der Technik eine Verbesserung der Korrelation der Meßwerte und eine wesentliche Verminderung des Achsenabschnitts beim Vergleich mit der HPLC-Referenzmethode erhalten wird.


Beispiel 3

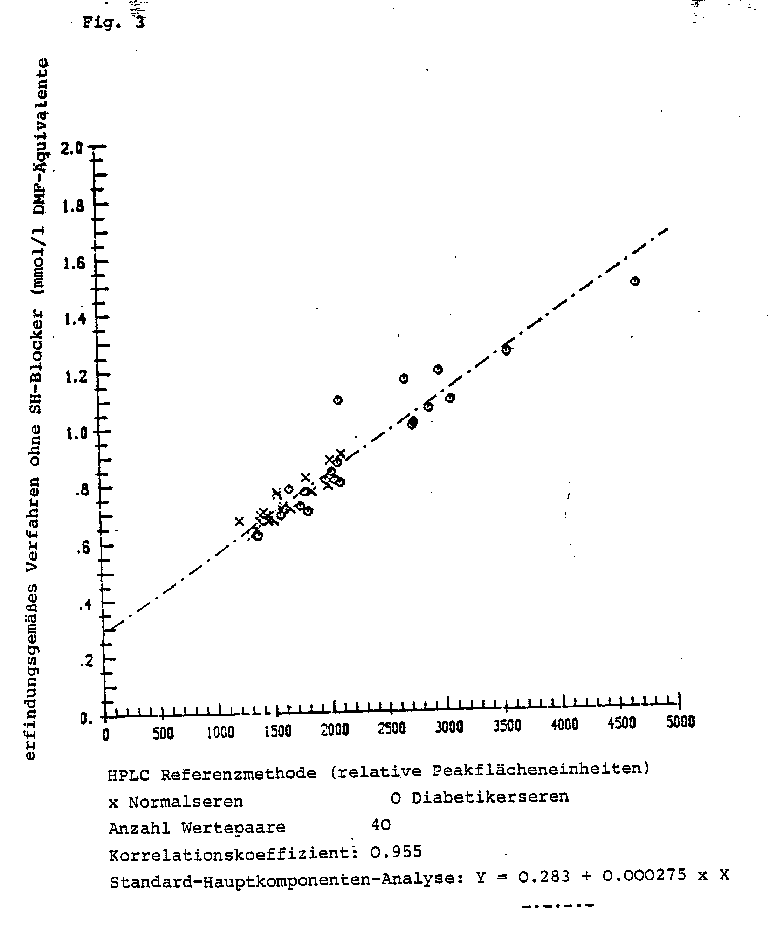

Bestimmung von Serumfructosamin wie in Beispiel 2, jedoch ohne Jodacetamid. In Fig. 3 sind die so erhaltenen Meßwerte gegen mit der Furosin/HPLC-Methode ermittelte aufgetragen.

Obwohl Fig. 3 gegenüber Fig. 1 einen wesentlich verminderten Achsenabschnitt zeigt und damit bereits eine wesentlich zuverlässigere Aussage des tatsächlichen Serumfructosamingehaltes erlaubt, ist einem Vergleich von Fig. 3 mit Fig. 2 der zusätzliche entstörende Einfluß des SH-Gruppen blockierenden Reagenzes deutlich zu entnehmen.


Beispiel 4

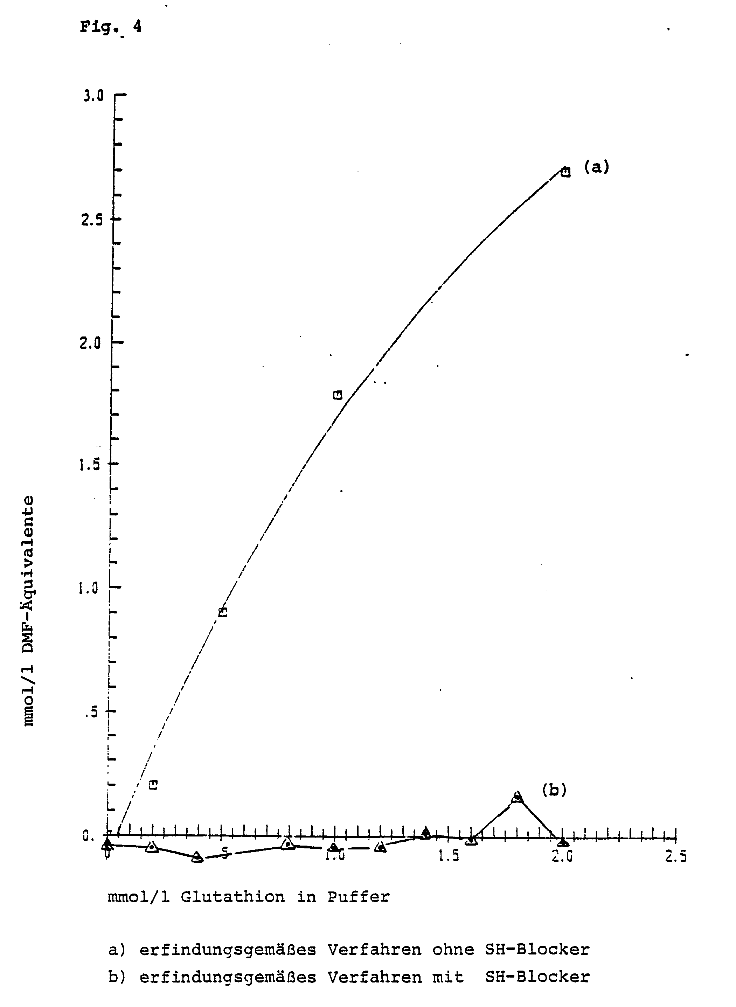

Der Einfluß von steigenden Mengen SH-Gruppen, beispielsweise von Glutathion, auf die Bestimmung von Serumfructosamin ist in Fig. 4 dargestellt.

In a) ist die Reagenzzusammensetzung wie in Beispiel 3,

in b) ist die Reagenzzusammensetzung wie in Beispiel 2.

Die Messung des Fructosamingehaltes erfolgt jeweils, wie in den entsprechenden Beispielen 2 und 3 beschrieben. Es ist festzustellen, daß eine SH-Gruppen-Blockierung die Störung zuverlässig beseitigt. Durch SH-Gruppen wird ohne Zusatz SH-Gruppen blockierender Reagenzien nichtenzymatisch glykosyliertes Protein vorgetäuscht.


**Ansprüche**

1. Verfahren zur spezifischen Bestimmung des Serumfructosamingehaltes in Blut oder von Blut abgeleiteten Proben durch Umsetzung mit einem geeigneten Farbreagenz und Messung der dabei bewirkten Farbänderung, wobei vor der Farbreaktion unspezifisch reduzierend wirkende oder/und trübungsverursachende Probenbestandteile beseitigt werden und anschließend bei einem pH-Wert zwischen pH 10 und 12 das Farbreagenz zugegeben wird,

dadurch gekennzeichnet, daß zur Beseitigung unspezifisch reduzierend wirkender Probenbestandteile die Probe bei ungefähr neutralem pH-Wert mit einem oder mehreren enzymatischen Oxidationsmitteln, gegebenenfalls zusammen mit Peroxidase oder/und Katalase sowie einer oder mehreren SH-Gruppen blockierenden Substanzen, behandelt wird.

2. Verfahren gemäß Anspruch 1, dadurch gekennzeichnet, daß als enzymatisches Oxidationsmittel Ascorbat-Oxidase, Bilirubin-Oxidase oder/und Uricase und als SH-Gruppen blockierende Substanzen Jodacetamid, Jodacetat, N-Ethylmaleinimid oder/und p-Hydroxymercuribenzoat eingesetzt werden.

3. Verfahren gemäß einem der Ansprüche 1 bis 2, dadurch gekennzeichnet, daß zusätzlich ein oder mehrere Detergentien zugesetzt werden.

4. Verfahren gemäß Anspruch 3, dadurch gekennzeichnet, daß als SH-Gruppen blockierende Substanz Jodacetamid und ein nichtionisches oder/und anionisches Detergenz zugesetzt wird.

5. Verfahren gemäß Anspruch 1, dadurch gekennzeichnet, daß zur Beseitigung trübungsverursachender Probenbestandteile die Probe mit Lipase, gegebenenfalls zusammen mit einem oder mehreren Detergentien oder/und Salzen starker Säuren behandelt wird.

6. Verfahren gemäß Anspruch 5, dadurch gekennzeichnet, daß als Detergentien anionische oder/und nichtionische Detergentien und als Salze starker Säuren Alkali- oder Erdalkalisalze von Salz- oder Schwefelsäure eingesetzt werden.

7. Verfahren gemäß einem der Ansprüche 1 - 6, dadurch gekennzeichnet, daß die Beseitigung unspezifisch reduzierend wirkender oder/und trübungsverursachender Probenbestandteile bei einem. pH-Wert in dem Bereich von pH 6 - 9 durchgeführt wird.

8. Verfahren gemäß Anspruch 1, dadurch gekennzeichnet, daß die das zu bestimmende Fructosamin enthaltende Probe nach der Vorinkubation zur Beseitigung unspezifisch reduzierend wirkender oder/und trübungsverursachender Probenbestandteile zur Umpufferung auf einen pH-Wert in dem Bereich von pH 10 bis 12 mit einem Puffer versetzt wird, der einen pH-Wert in dem Bereich von pH 10,5 bis 12,5 aufweist.

9. Verfahren gemäß Anspruch 8, dadurch gekennzeichnet, daß gleichzeitig mit dem Puffer, der einen pH-Wert im Bereich von 10,5 bis 12,5 aufweist, das Farbreagenz zugegeben wird.

10. Reagenzkombination zur spezifischen Bestimmung des Serumfructosamingehaltes in Blut oder von Blut abgeleiteten Proben, bestehend aus einem Reagenz zur Beseitigung unspezifisch reduzierend wirkender oder/und trübungsverursachender Probenbestandteile,
einem Umpufferungsreagenz mit einem Puffer, der einen alkalischen pH-Wert aufweist und
einem Farbreagenz zum Nachweis des Fructosamins,
dadurch gekennzeichnet, daß das Reagenz zur Beseitigung unspezifisch reduzierend wirkender oder/und trübungsverursachender Probenbestandteile in einem Puffer mit annähernd neutralem pH-Wert ein oder mehrere enzymatische Oxidationsmittel sowie gegebenenfalls Peroxidase oder/und Katalase,
oder/und Lipase sowie gegebenenfalls ein oder mehrere Detergentien oder/und Salze starker Säuren sowie eine oder mehrere SH-Gruppen blockierende Substanzen,
gegebenenfalls übliche Zusatzstoffe enthält.

11. Reagenzkombination gemäß Anspruch 10, dadurch gekennzeichnet, daß sie als enzymatische Oxidationsmittel Ascorbat-Oxidase, Bilirubin-Oxidase oder/und Uricase and als SH-Gruppen blockierende Substanzen Jodacetamid, Jodacetat, N-Ethylmaleinimid oder/und p-Hydroxymercuribenzoat enthält.

12. Reagenzkombination gemäß Anspruch 10, dadurch gekennzeichnet, daß sie als Detergentien kationische, anionische oder/und nichtionische Detergentien und als Salze starker Säuren Alkali- oder Erdalkalisalze von Salz- oder Schwefelsäure enthält.

13. Reagenzkombination gemäß Anspruch 10, dadurch gekennzeichnet, daß sie als SH-Gruppen blockierende Substanz Jodacetamid und ein nichtionisches oder/und anionisches Detergenz enthält.

14. Reagenzkombination gemäß Anspruch 10, dadurch gekennzeichnet, daß der Puffer mit dem Reagenz zur Beseitigung unspezifisch reduzierend wirkender oder/und trübungsverursachender Probenbestandteile einen pH-Wert in dem Bereich von 6 bis 9 aufweist.

15. Reagenzkombination gemäß anspruch 10, dadurch gekennzeichnet, daß der Puffer des Umpufferungsreagenzes einen pH-Wert im Bereich von pH 10,5 bis 12,5 aufweist.

16. Verwendung eines Reagenzes zur Beseitigung unspezifisch reduzierend wirkender oder/und trübungsverursachender Probenbestandteile gemäß Anspruch 10 zur Bestimmung des Serumfructosamingehaltes in Blut oder in von Blut abgeleiteten Proben.

**Fig. 1**

HPLC Referenzmethode (relative Peakflächeneinheiten)

x Normalseren          O Diabetikerseren

Anzahl Wertepaare:        33

Korrelationskoeffizient: 0.945

Standard-Hauptkomponenten-Analyse: Y = 1.41 + 0.000748 x X

Fig. 2

HPLC-Referenzmethode (relative Peakflächeneinheiten)

x Normalseren                    O Diabetikerseren

Anzahl Wertepaare:              33

Korrelationskoeffizient: 0.973

Standard-Hauptkomponenten-Analyse: Y = 0.0706 + 0.000348 x X

Fig. 3

Fig. 3 — Korrelationsdiagramm: erfindungsgemäßes Verfahren ohne SH-Blocker (mmol/l DMF-Äquivalente) gegen HPLC Referenzmethode (relative Peakflächeneinheiten)

HPLC Referenzmethode (relative Peakflächeneinheiten)

x Normalseren         O Diabetikerseren

Anzahl Wertepaare    40

Korrelationskoeffizient: 0.955

Standard-Hauptkomponenten-Analyse: $Y = 0.283 + 0.000275 \times X$

Fig. 4

a) erfindungsgemäßes Verfahren ohne SH-Blocker

b) erfindungsgemäßes Verfahren mit  SH-Blocker